# EUROPEAN PATENT SPECIFICATION

(11) **EP 0 642 777 B1**
(45) Date of publication and mention of the grant of the patent: **03.11.1999**
(21) Application number: 94113623.6
(22) Date of filing: 31.08.1994
(51) Int. Cl.: A61F 13/10

(54) **Arm support garment**
Armbandage
Bandage pour bras

(30) Priority: 14.09.1993 JP 22923893
(43) Date of publication of application: 15.03.1995
(73) Proprietor: WACOAL CORP., Kyoto-shi, Kyoto-Fu 601 (JP)
(72) Inventor: Iwata, Tetsuya, No. 409, Detom-1 Nishijin P.3, 872, Kamigyo-ku, Kyoto-shi, Kyoto-fu, 602 (JP)
(74) Representative: VOSSIUS & PARTNER

(56) References cited:
- EP-A- 0 275 613
- EP-A- 0 519 135
- FR-A- 2 358 872
- GB-A- 2 242 818
- US-A- 3 934 583

## Description

The present invention relates to arm support garments, and more specifically to an arm support garment which is applied closely onto the surface of human forearms primarily to prevent muscle fatigue therein.

These days it is well known that concentrated long-time use of forearms by sports or training activities such as baseball, tennis, bicycling, motorcycling contributes not only to accumulating factors of fatigue including lactic acid in the vicinity of the muscles in use to cause fatigue but also to muscle disorders such as muscle pain. In order to prevent such muscle pain or to permit continued sports practice while protecting the damaged part even when one gets muscle ache, sometimes taping treatment is applied on the muscle region to support the area by compressing strongly around the region to limit the muscle stretch and movements of the muscle. Also supporters of a tube-like shape made of a stretchable relatively thick pile fabric or neoprene are often used to compress human body parts, such as arms, inwardly from the circumference.

However, the above-mentioned conventional taping method requires skill and if the treatment is applied inadequately, it not only fails to accomplish the prevention or treatment purpose but also can produce an adverse effect on the muscle area such as disturbing physical movements, increased susceptibility to blood circulation disorders or nervous disorders. Therefore the taping method can be applied only by those who are skilled in the method but not by those who are not.

As for the latter supporter method, such supporters can be worn easily but they simply compress strongly the entire region to be protected. Thus, they compress the muscle groups' motor points significantly and also prevent proper blood circulation and cause discomfort. This may result in blood circulation disorders providing little effect to alleviate the muscle fatigue. Moreover, because the supporters are made by thick rubber materials such as neoprene or thick pile fabric, they have such shortcomings as disturbing arms' movements or becoming stuffy due to their bad breathability.

EP-A-0 519 135 discloses wearing articles or clothes with a taping function and in particular an elbow supporter in which a heavily-stretchable cloth piece is provided so as to be disposed on the upper arm portion and the front arm portion.

GB-A-2 242 818 discloses covering articles, e.g. a tennis elbow outfit, and a method of making-up thereof.

It is an object of the present invention to provide an arm support garment which is applied closely and compressingly on the surface of a human arm to prevent disorders caused by muscle fatigue and can be easily put on adequately by anybody without causing blood circulation disorders or interrupting muscle contraction. Therefore, the support garment of the present invention does not disturb the arms' smooth movements in sports activity but is able to follow the wearer's arm's twist movements (pronation, supination) comparatively well and is capable of relieving and preventing the arms' fatigue.

The present invention relates to an arm support garment of tube-like shaped stretchable material applied on arms' area, wherein a stretchable material having a comparatively weak straining force is applied to the portion which in use covers the motor points of each of the flexor muscle group and the extensor muscle group extending generally in the longitudinal direction of the forearm parts; and wherein a stretchable material having a comparatively strong straining force is applied to the portion which in use covers the vicinity of the both longitudinal sides of said motor point of the flexor muscle group and the extensor muscle group.

It is preferable that in an arm support garment in the present invention, the whole portion comprises a stretchable material of a comparatively weak straining force and a stretchable material of a comparatively strong straining force is superimposed on the portion which in use covers the vicinity of the both longitudinal sides of the motor point of the flexor muscle group and the extensor muscle group, extending in the longitudinal direction of forearm parts.

It is also preferable that in an arm support garment in the present invention, a stretchable knit fabric or a stretchable woven fabric is used as the stretchable material.

It is also preferable that in the arm support garment in the present invention, the stretchable material of a comparatively weak straining force is a stretchable material having a straining force of 0,294 - 0,784 N (30 - 80 gf) in width direction, and the stretchable material of a comparatively strong straining force is a stretchable material having a straining force of 0,98 - 2,94 N (100 - 300 gf) in width direction.

It is also preferable that in the arm support garment in the present invention, the stretchable material of a comparatively weak straining force is all ways stretch tricot.

It is also preferable that in the arm support garment in the present invention, the stretchable material of a comparatively strong straining force is spandex power net.

It is also preferable that the arm support garment in the present invention is further provided with a strap portion for inserting of fingers at the end of the wrist side.

It is also preferable that the arm support garment in the present invention is further provided with a protection piece in the vicinity of the wrist to prevent overextension.

An arm support garment in the present invention is a tube-shaped arm support garment made of a stretchable material, to be worn on the arm region. The garment includes a stretchable material of a comparatively weak straining force to cover the motor points of each of the flexor muscle group and the extensor muscle group extending generally along the longitudinal direction of forearms. That is, the motor points where the muscles of primary muscle groups stretch and contract most for moving the arm are compressed lightly without interferring with these muscle movements while stretching and contracting. The garment includes a stretchable material of a comparatively strong straining force in the vicinity of the both longitudinal sides of said flexor muscle group and/or the extensor muscle group extending generally in the longitudinal direction of forearms to supportingly compress said muscle groups from their side portions without suppressing the motor points which pursue the primary function of muscles in the forearms. The compression narrows the diameters of blood vessels and lymphatics to accelerate the blood and lymph circulation --just like narrowing a water hose diameter increases speed of the water discharge -- to promote removal of factors of fatigue such as lactic acid and alleviate or prevent muscle fatigue and consequently to prevent disorders caused by accumulated muscle fatigue without interrupting smooth movement of the arms or causing discomfort. Moreover due to the construction not having stretchable material with a comparatively strong straining force along the middle portion of the motor points, the garment would not cause blood disorders or muscle movement disorders. Besides because the shape of the garment is tube-like and the regions of a relatively weak straining force and the regions of a relatively strong straining force are determined and settled in the specific regions, anybody can easily wear the garment properly.

Since a preferable embodiment of the arm support garments of the present invention has a tube-like shape and comprises a stretchable material of a comparatively weak straining force in the entire region and with a stretchable material of a comparatively strong straining force superimposed along the vicinity of the both longitudinal sides of the motor point of the flexor muscle group and the extensor muscle group extending generally along the longitudinal direction of forearms, the garment can be manufactured simply by superimposing a stretchable material of a relatively strong straining force onto specific portions of the garment comprised of a stretchable material of a relatively weak straining force without complicated design or the need for a production process which requires seaming fabrics of a stretchable material having a relatively weak straining force and fabrics of a stretchable material having a relatively strong straining force alternately.

Since a preferable embodiment of the arm support garments of the present invention utilizes a stretchable knit fabric or a stretchable woven fabric as the stretchable material, a garment with thinness, lightness, better fittability, excellent ventilation, and comfortable feeling can be provided.

Since a preferable embodiment of the arm support garments of the present invention utilizes a stretchable material of a comparatively weak straining force in the direction of the width of 0,294 - 0,784 N (30 - 80 gf) and a stretchable material of a comparatively strong straining force in the direction of the width of 0,98 - 2,94 N (100 - 300 gf), the garment pursues well-balanced functions of physical movements without interfering with arms' movements, and relief and prevention of fatigue without blood circulation disorders. Moreover, because the stretchable material of a relatively weak straining force is of a straining force of 0,294 - 0,784 N (30- 80 gf) in the direction of the width, a garment which has soft fittability, better feeling and ventilation, is easy to put on due to its easiness to expand, and has excellent fittability regardless of the size of the arms of the wearer can be provided.

In a preferable embodiment of the arm support garments in this invention, all ways stretch tricot is used as a stretchable material of a comparatively weak straining force. The all ways stretch tricot is a thin knit fabric unlike pile fabric or neoprene used to the conventional supporters, and a garment which has soft fittability and lightness, better feeling and ventilation, is easy to put on due to its easiness to expand, and has excellent fittability regardless of the size of the arms of the wearer.

In a preferable embodiment of the arm support garments in the present invention, spandex power net is used as a stretchable material of a comparatively strong straining force. The spandex power net is a thin knit fabric with sufficiently strong stretching power unlike pile fabric or neoprene used to the conventional supporters, and a garment with good ventilation without stuffiness, with lightness, without interruption of arms' movements, and with an aesthetically pleasing wearing silhouette which is accomplished by its fittability and thinness, can be provided.

A preferable embodiment of the arm support garments in this invention is further provided with a strap portion to insert the wearer's fingers at the end of the wrist side, and thus with such an easy operation to inserting fingers into the strap, the garment can easily be put on and oriented properly and also would not slide up toward the elbow even in sports activities. The garment also can follow the arm's twisting movements to allow the stretchable material to stay on the targeted muscle regions even when the wearer twists the arm, and consequently can be accommodated to the muscle position's change.

A preferable embodiment of the arm support garments in the present invention is further provided with a protection piece in the vicinity of the wrist to prevent overextension. Thus, the garment can further reduce the chances of overextension or sprain of the wrist.

### BRIEF DESCRIPTION OF THE DRAWINGS

FIG. 1 illustrates a development of the parts of the arm support garment as one embodiment in this invention.
FIG. 2 is a side view of this embodiment of the present invention of arm support garments with a strap hole to insert the pollex facing to the upper margin of the drawing.
FIG. 3 is a side view of the same embodiment of the present invention of arm support garments shown in FIG. 2 viewed from the opposite side.
FIG. 4 is a plan view of the same embodiment of the present invention of arm support garments shown in FIG. 2 with its strap hole to insert pollex facing perpendicular to the surface of the drawing and viewed from the above.
FIG. 5 is a plan view of the same embodiment of the present invention of arm support garments shown in FIG. 4 viewed from the opposite side of FIG. 4.
FIG. 6 is a perspective view of the same embodiment of the present invention of arm support garments shown in FIGs. 2-5 viewed in use onto the backside of the wearer's hand.
FIG. 7 is a perspective view of the same embodiment of the present invention of arm support garments shown in FIGs. 2-5 viewed in use onto the palm side of the wearer.
FIG. 8 illustrates a development of the main part of an arm support garment of another embodiment of the present invention.
FIG. 9 illustrates a development of the main part of an arm support garment of another embodiment of the present invention.
FIG. 10 illustrates a development of the main part of an arm support garment of another embodiment of the present invention.
FIG. 11 is a Schematic drawing illustrating positions of primary muscles of a human right forearm with its palm up.
FIG. 12 is a schematic drawing illustrating positions of primary muscles of a human right forearm with its back of the hand up.

It can be easily understood that concrete embodiments of the arm support garments of the present invention can be any kind of the garment as far as the embodiments do not spoil the object or the nature of this invention. That is, the garments can be the ones which are applied onto arm portions, in particular to forearm portions, have a tube-like shape, and are ride of a stretchable fabric to compress the surface of human arms. The garments have a stretchable material of a relatively weak straining force in the portions which in use cover the motor points of the flexor muscle group and the extensor muscle group extending along the longitudinal direction of forearms, and have a stretchable material of a relatively strong straining force in the vicinity of the both longitudinal sides of said motor point of the flexor muscle group and the extensor muscle group. As for the length of the garment, the garment can be a shorter one not reaching to the wrist or a shorter one not reaching to the elbow, or can be a Longer one reaching to the upper arm or connected to a garment for upper body such as the sleeve of a coat.

Some representative examples of the present invention are described in the drawings. As mentioned before, the present invention is not limited to the embodiments described herein.

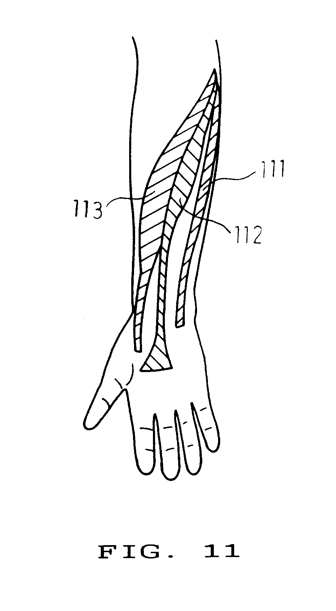

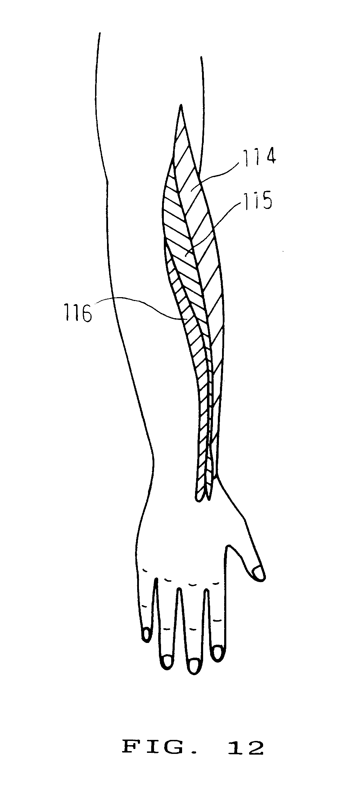

The forearm muscles described in the present invention are outlined in the FIGs. 11 and 12.

FIG. 11 illustrates the positions of the primary muscles of the right arm with the palm up. The numeral 111 represents the flexor carpi ulnaris, 112 the palmaris longus 113 the flexor carpi radialis. The "flexor muscle group" is the general term for these three muscles in the present invention.

FIG. 12 illustrates the position of primary muscles of a right arm with the back of the hand up. The numeral 114 represents the brachioradialis, 115 the extensor carpi radialis longus, 116 the extensor carpi radialis brevis. The "extensor muscle group" is a general term for these three muscles in the present invention.

The flexor muscle group and the extensor muscle group are the essential muscles in the forearm portion. In this invention it is more convenient and easier to deal with the flexor carpi ulnaris 111, the palmaris longus 112, the flexor carpi radialis 113 like one muscle rather than to deal with them individually. Thus the three muscles are called the flexor muscle group herein as if they are one muscle. The same can be said for the extensor muscle group. Therefore the motor points of the flexor muscle group or the extensor muscle group represent the region where the muscles are relatively thicker in width, excluding the vicinity of tendons of the muscles when they are treated as one muscle. "The vicinity of the both longitudinal sides of the flexor muscle group along the longitudinal direction of forearm parts" is defined as the area along the both sides of the entire flexor muscle group in the longitudinal direction of the forearm parts when the flexor muscle group is viewed as one muscle, and not the vicinity of the sides of the individual muscles as the flexor carpi ulnaris 111, the palmaris longus 112, the flexor carpi radialis 113. This definition can be equivalently applied to the description of "the vicinity of both sides of the extensor muscle group in the longitudinal direction of the forearm parts".

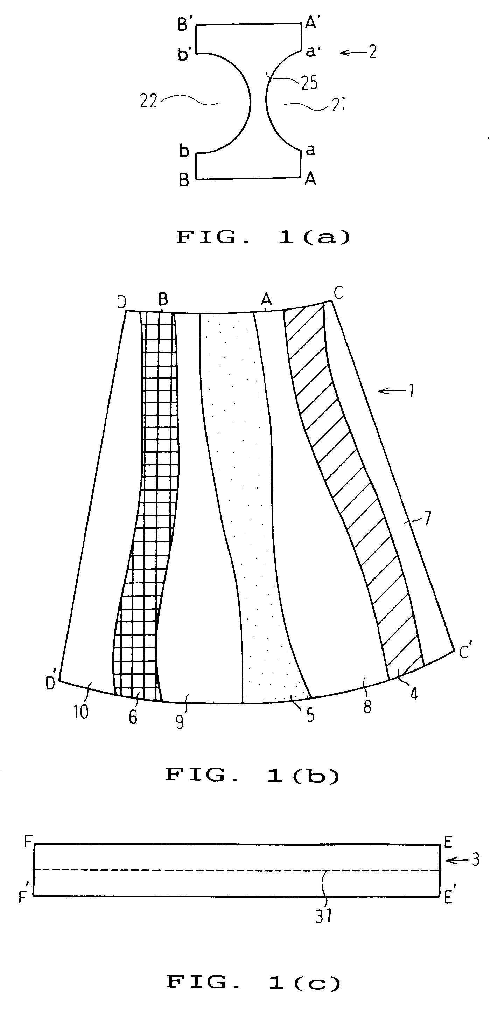

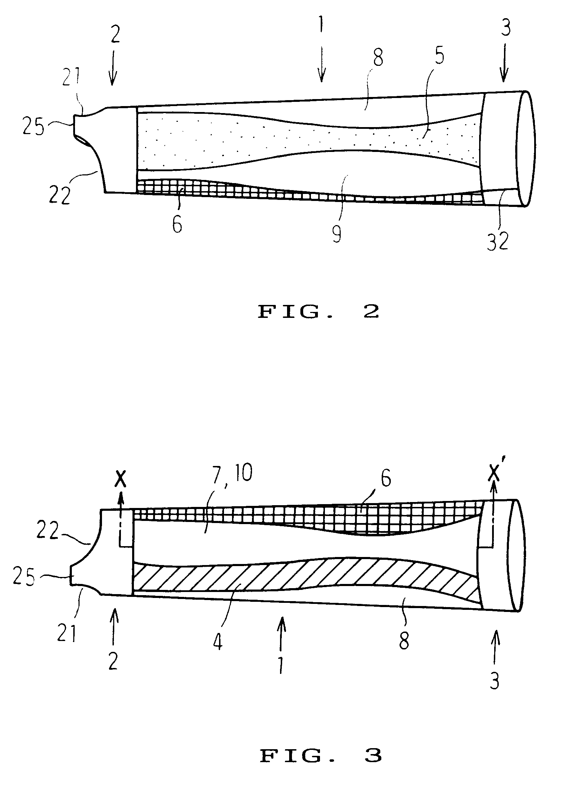

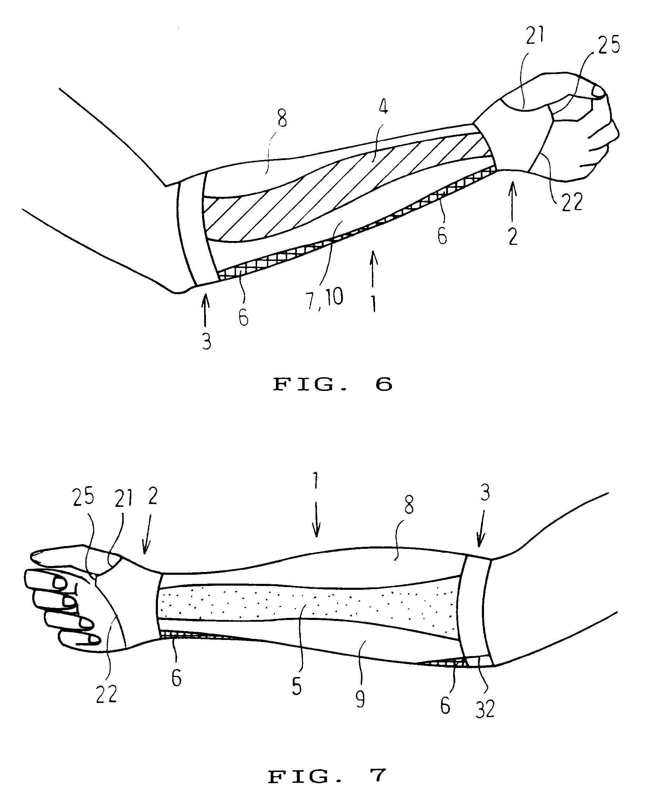

FIG. 1 illustrates a development of the parts of the arm support garment as one embodiment in this invention. The development illustrates an embodiment of the arm support garment in the present invention having a strap to insert fingers at the end of the wrist side and a ring-shaped edge piece at the opposite end. In the drawing, (a) illustrates the development of the strap to insert fingers, (b) illustrates the development of the main part of the arm support garment, (c) illustrates the development of the edge piece. Plan views of the finished product comprising those parts stitched together, observed from several directions, are illustrated in FIGs. 2-5 and views in use are illustrated in FIGs. 6-7.

FIGs. 2-7 illustrate only the right arm portion of the arm support garment and the left arm portion is not described herein. However, because tie positions of the flexor muscle group or the extensor muscle group are plane-symmetrical about the plane which divides human body into the right and the left portion symmetrically in half, in other words bilaterally symmetrical, the left hand portion can be understood to be the one bilaterally symmetrical to the right hand portion.

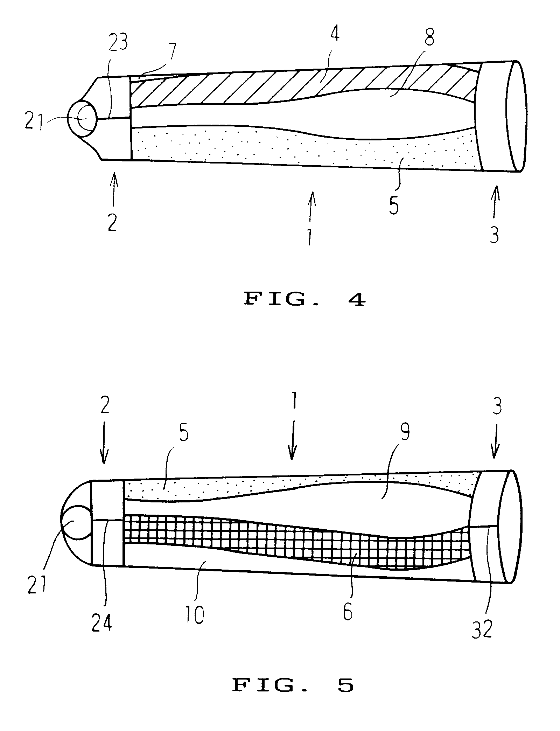

FIG. 2 is a side view of the present invention of the arm support garments with the strap hole 21 to insert the pollex facing the upper margin of the drawing, in other words, the drawing of the garment with the palm up viewed from the above. FIG. 3 is a side view from the opposite side with the strap hole 21 to insert the pollex facing the lower margin of the drawing, in other words, the drawing of the garment with the back of the band up viewed from the above. FIG. 4 is a plan view with the strap hole 21 to insert the pollex facing perpendicular to the surface of the drawing and toward the viewers observed from above, that is, with the palm of the pollex side upward and perpendicular to the surface and observed from above. FIG. 5 is a plan view observed from the opposite side of FIG. 4 with the palm of the pollex side downward and perpendicular to the surface and observed from the above, that is, a drawing observed from the little finger side.

FIG. 6 illustrates the arm support garment described in FIGs. 2-5 in use and observed from the back of the hand, and FIG. 7 illustrates the arm support garment described in FIGs. 2-5 in use and observed from the palm side.

As described in the development of the main part of the arm support garment in FIG. 1 (b), in this example, the whole portion of the main part 1 comprises a stretchable material of a comparative weak stretchable force such as all ways stretch tricot. Portion 8, which in use covers mainly over the motor points of the extensor muscle group thus comprises a stretchable material of a comparatively weak stretchable force such as all ways stretch tricot, and portion 9 which in use covers mainly over the motor points of the flexor muscle group comprises a stretchable material of a comparatively weak stretchable force such as all ways stretch tricot. Portions 4, 5, 6 further comprise a stretchable material of a comparatively strong stretchable force, such as spandex power net, which is stitched and lined to the main part 1 in the vicinity of the both longitudinal sides of the motor points of the extensor muscle group and the flexor muscle group respectively. The portions 7 and 10 in this embodiment are part of the main part 1 which comprises a stretchable material of a comparatively weak straining force though it is not particularly limited. The main part 1 shown by the development in FIG. 1 forms a tube-like shape (a tube-like shape with its wrist end slightly narrower) with the C-C' line and D-D' line in the FIG. 1 (b) connected together. The structure is apparent from the FIGs. 2-7. Because the FIG. 1 (b) is the development, C-C' line and D-D' line are described as the edges of the main part 1 for convenience and the lines are not limited to be the edges of the main part 1, since it is also possible to knit the main part cylindrically and still have the straining force of each portion as specified.

To form the part described in FIG. 1 (b) cylindrically by seaming, it is possible to seam at the lines of C-C' and D-D'. However, it is preferable to seam at a border line or at two border lines or more between a portion made of a stretchable material of a relatively weak straining force and a portion made of a stretchable material of a relatively strong straining force, such as 7 and 4, 4 and 8, 8 and 5, 5 and 9, 9 and 6 or 6 and 10 for better appearance.

In this embodiment, the whole portion of the main part 1 comprises a stretchable material of a relatively weak straining force, e.g. all ways stretch tricot knit fabric (having a straining force in the direction of the width of 0,372 N (38 gf), dyed in green) and the portion 4, 5, 6 along the vicinity of the longitudinal sides of the motor points of the flexor muscle group and the extensor muscle group comprises a stretchable material of a relatively strong straining force, e.g. spandex power net (having a straining force in the direction of the width of 1,25 N (128 gf), dyed in white) lined and superimposed to said main part. It is also possible to form the portions 7, 8, 9, 10 made of a stretchable material of a relatively weak straining force and the portions 4, 5, 6 made of a stretchable material of a relatively strong straining force by separate pieces and seam them at the specified parts to farm a tube-like shape. In the seaming, it is possible to form a dart in the portion where such dart is desired. For example, it is preferable to seam with a dart to follow the shape of such parts as the vicinity of the portion where the width of the muscle becomes thinner abruptly or the curved portion of an elbow in the case when the garment extends in use toward the upper arm via the elbow.

To superimpose a stretchable material of a relatively strong straining force to a stretchable material of a relatively weak straining force, it is also possible to laminate from the outer surface, or to further laminate a stretchable material of a relatively weak straining force on the stretchable material of a relatively strong straining force laminated from the outer surface to form a triple-layer structure if necessary.

Also it is possible to comprise a whole portion of the main part 1 with a stretchable material of a relatively weak straining force and strengthen the straining force in the vicinity of the longer sides of the motor points of the flexor muscle group and the extensor muscle group respectively by such methods as adhering a film or a net of elastomer resin, or coating with an elastic resin solution or emulsion or applying a resin treatment.

If the purpose of a wearer requires, it is also possible to apply a stretchable material of a relatively strong straining force on the portion along the vicinity of the longitudinal sides of the motor points of either the flexor muscle group or the extensor muscle group only. In the case where a stretchable material of a relatively strong straining force is to be applied only on the portion along the vicinity of the longitudinal sides of the motor points of the flexor muscle group, the portion 4 should comprise a stretchable material of a relatively weak straining force. On the contrary, if a stretchable material of a relatively strong straining force is to be applied only on the portion along the vicinity of the longitudianl sides of the motor points of the extensor muscle group, the portion 6 should comprise a stretchable material of a relatively weak straining force. However, it is preferable to apply a stretchable material of a relatively strong straining force on the portion in the vicinity of the both longitudinal sides of the motor points of both the flexor muscle group and the extensor muscle group.

The above mentioned technique can also be applied to the other embodiments later described.

FIG. 1 (c) illustrates a development of a ring-shaped edge piece which is to be attached to a main part 1 at the end opposite to the wrist, namely the C'-D' line. The edge piece will be folded over the line 31, namely putting point F to point F', point E to point E' respectively to be doubled up and then to be attached to the line C'-D'. The appearance of the edge piece 3 attached to the main part 1 can be observed in FIGs. 2-7.

Such edge piece 3 is not always necessary, but it is preferable to have a ring-shaped edge piece to minimize the sliding-up of the edge portion toward the wrist of the arm support garment of the present invention in use. The material of the edge piece varies depending on the position of the edge piece and is not limited particularly, but it is preferable to use a stretchable material of a comparatively moderate straining force not causing disorders even when it covers a part of motor points so as not to interfere the purpose of the present invention.

A strap portion will be described referring to the drawings of finished products in FIGs. 2-7 first, because it would facilitate understanding rather than referring to the development in FIG. 1 (a) first.

FIGs. 2-5 and FIGs. 6, 7 illustrate that an arm support garment in this embodiment has a strap 2 to insert fingers at the end of the wrist side. The strap 2 in this embodiment has two holes, namely a strap hole 21 to insert the pollex and a strap hole 22 to insert the other fingers, and a stopper 25 is formed at the portion between the pollex and index finger.

Though a simple operation of inserting the pollex in strap hole 21 and the other fingers in strap hole 22, the structure enables the garment to be worn with a stretchable material of a relatively weak straining force and a stretchable material of a relatively strong straining force placed in the specified portion and oriented in the proper direction. The stopper 25 of the strap hooks the portion between the pollex and index finger to prevent sliding-up of the arm support garment toward the elbow in motion. Besides, even when position of muscles changes by the wearer's arm-twisting motion, the arm support garments in this invention follow the arm's twisting motion sufficiently to maintain the stretchable material on the targeted position.

The embodiment has a strap with two strap holes, namely the strap hole 21 to insert the pollex and the strap hole 22 to insert the other fingers, however, it can be easily understood that the shape of the strap is not limited to this type but any embodiment which can accomplish the above mentioned purpose of a strap, for example a type having holes for each fingers, can be employed. It is preferable to have a strap but a strap is not always required. It is possible to provide a ring-shaped edge piece as mentioned above instead of the strap at the end of the wrist side, namely at C-D line of FIG. 1 (b). Such technique can be applied to the other embodiments.

The following can be understood by referring to the illustration of development of a strap 2 in FIG. 1 (a). By seaming the A-a line and A'-a' line by such measures as stitching, point a and point a' will be attached to form a strap hole 21 to insert the pollex. Likewise by seaming the B-b line and B'-b' line by such measures as stitching, point b and point b' will be attached to form a strap hole 22 to insert the other fingers. Subsequently the points A and A', B and B' will be connected together so that lines A-B and B'-A' will form a loop. Then the line A-B shown in FIG. 1 (a) will be attached to the line A-B shown in FIG. 1 (b), and the line B'-A' shown in FIG. 1 (a) will be attached to the line B-D-C-A in FIG. 1 (b) to form a strap 2 illustrated in FIGs. 2-7.

As already described in the explanation of FIG. 1 (b), in FIGs. 2-7, the main part 1 of the arm support garment comprises a stretchable material of a comparatively weak straining force such as all ways stretch tricot, portion 8 which in use covers mainly the motor points of the extensor muscle group comprises a stretchable material of a comparatively weak stretchable force such as all ways stretch tricot, and portion 9 which in use covers mainly the motor points of the flexor muscle group comprises a stretchable material of a comparatively weak stretchable force such as all ways stretch tricot, whereas portions 4, 5, 6 comprise a stretchable material of a comparatively strong stretchable force such as spandex power net, stitched and lined to the main part 1 along the vicinity of the longitudinal sides of the motor points of both the extensor muscle group and the flexor muscle group respectively. The illustration of the development in FIG. 1 is the development of the garment in FIG. 3 cut along the line X-X' and spread out. In FIG. 1 (b), portions numbered 7, 10 are illustrated as the right and left edges of the development, but in FIGs. 2-7 (in particular FIGs. 7, 6) portions numbered 7, 10 are described integrally. The portions 7, 10 are not particularly limited but in this embodiment are described as a part of the main body 1 which comprises a stretchable material of a relatively weak straining force such as all ways stretch tricot. In general, except for the parts which in use lie in the vicinity of the longitudinal sides of the motor point of the flexor muscle group and/or the extensor muscle group and are provided with a stretchable material of a relatively strong straining force, the other parts of the garment are provided with a stretchable material of a relatively weak straining force.

This embodiment of the arm support garment supports muscle groups from the sides to promote blood circulation in the muscles without compressing the motor points of muscles which have main functions in movement, and facilitates the removal of factors of fatigue such as lactic acid without hindrance of the arm's smooth movements or wearing discomfort. Thus, the arm support garment can prevent disorders caused by muscle fatigue. In particular, garments having a stretchable material with a relatively strong straining force in the portion along the vicinity of the longitudinal sides of the motor points of both the flexor muscle group and the extensor muscle group are preferable, because factors of fatigue are removed from the arms faster.

By using all ways stretch tricot as a stretchable material of a relatively weak straining force and spandex power net as a stretchable material of a relatively strong straining force, an arm support garment which is thinner than conventional supporters utilizing such materials as pile fabric or neoprene, light and capable of moving arms smoothly, excellently fittable, with comfortable feeling and good ventilation, easy to put on as it can be expanded easily, and capable of being adjusted to the wearer's arm's size can be provided. Also, because the arm support garment is provided with a strap 2 to insert fingers at the end of the wrist side, the garment can be easily worn on the specified portion of the arm with proper orientation through the simple operation of inserting fingers into the specified portion of the strap, and sliding-up toward the elbow can be minimized even in physical movements due to the structure in which the strap hooks between the pollex and the index finger. Moreover because the arm support garment is provided with a ring-shaped edge piece at the end opposite to the wrist end, the garment with minimum sliding-up toward the wrist end can be provided. Even when the wearer pronates or supinates an arm wearing the arm support garment and the position of muscles changes subsequently, the arm support garment in the present invention follows the arm twist movements to maintain the specified stretchable material on the intended muscle portions since it is being made of a stretchable material of a tube-shape, is fittable to the arm and has a strap.

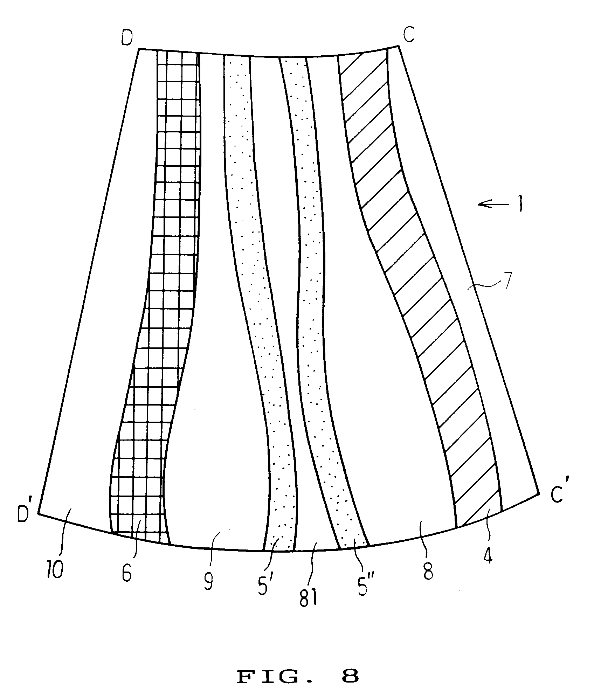

FIGs. 8-10 illustrate developments of other embodiments of the same portion as FIG. 1 (b).

In the embodiment of FIG. 8, the portion made of a stretchable material of a relatively strong straining force shown in FIG. 1 (b) is devided in two portions numbered 5' and 5''. The portion numbered 5' is of a stretchable material of a relatively strong straining force and extends along the vicinity of a longitudinal side of the motor points of the flexor muscle group and is slightly curved toward the flexor muscle group as compared with FIG. 1 (b). The portion numbered 5'' is of a stretchable material of a relatively strong straining force and extends along the vicinity of a longitudinal side of the motor points of the extensor muscle group and is slightly curved toward the extensor muscle group as compared with FIG. 1 (b). A portion 81 of a relatively weak straining force exists between these portions of a relatively strong straining force 5', 5''. The other parts are the same as FIG. 1 (b).

As described in FIG. 1, a stretchable material of a relatively strong straining force can be applied only to a portion along the vicinity of the longitudinal sides of the motor point of either the flexor muscle group or the extensor muscle group by either leaving the portions numbered 6 and 5' only with a stretchable material of a relatively weak straining force, or by leaving the portions numbered 5'' and 4 only with a stretchable material of a relatively weak straining force depending on the wearer's purpose. Such technique can be similarly applied to the following embodiments, and therefore further explanation will be omitted.

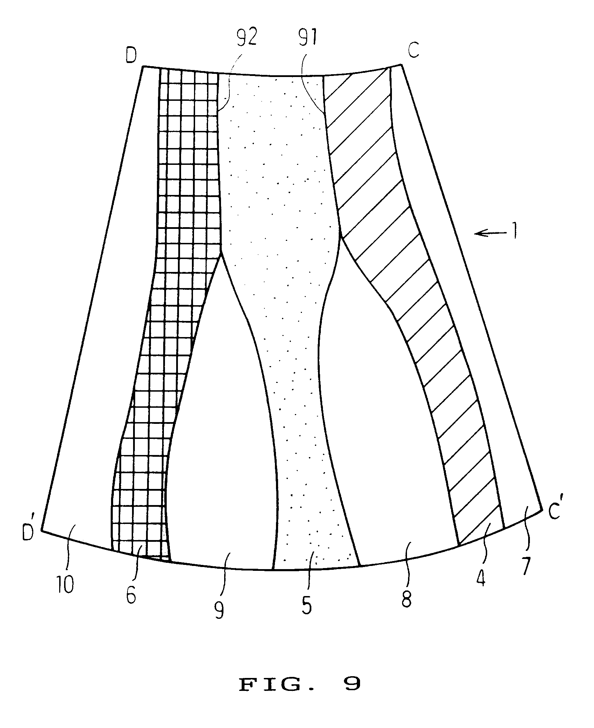

In the embodiment in FIG. 9, the portions of a relatively strong straining force numbered 4 and 5 shown in FIG. 1 (b) are seamed integrally at the seam part 91 at the wrist portion, thereby avoiding the relatively thick portion of the motor points of the extensor muscle group in that area, and similarly, the portions of a relatively strong straining force numbered 5 and 6 are seamed integrally at the seam part 92 at the wrist portion, thereby avoiding the relatively thick portion of the motor points of the flexor muscle group in that area. In the vicinity of a wrist, there are comparatively many tendons and the region is relatively hard so that applying a relatively strong straining force to such portions can promote the removal of factors of fatigue without such problems as the garment cutting into the muscles, deterioration of wearing comfort, and blood circulation disorders. Seam part 91 and/or seam part 92 can be formed from an integral fabric not forming seam parts.

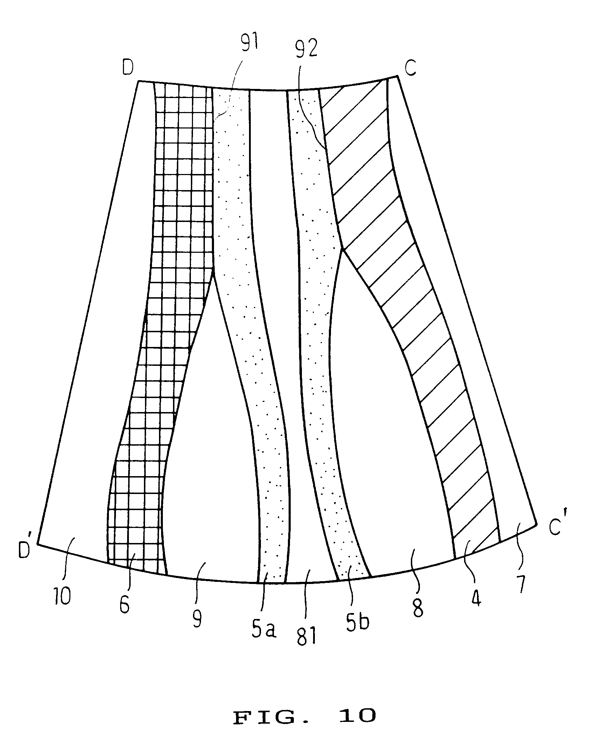

FIG. 10 illustrates an embodiment similar to FIG. 9 except the portion of a relatively strong straining force numbered 5 is divided in two portions numbered 5a and 5b. Compared to FIG. 9, portion 5a curves toward the flexor muscle group and portion 5b curves toward the extensor muscle group slightly more. A portion with a comparatively weak straining force numbered 81 exists between the portions 5a, 5b of a relatively strong straining force. The other parts are same as FIG. 9. Similar to FIG. 9, seam part 91 and/or seam part 92 can be formed from an integral fabric without forming seam parts.

Assuming that the same materials are employed for each component for the embodiments shown in FIGs. 1, 8, 9, 10, the compressing force in the portion around the wrist will be stronger in the ascending order of FIG. 8, FIG. 1, FIG. 10, FIG. 9.

Even in the same embodiment, straining force of a desired grade can be obtained by using a material of a greater straining force or of a less straining force. Or even when using the same kind of material, for example spandex power net, straining force of a desired grade can be obtained by controlling the amount or thickness of the fibers used therein, especially elastic fibers such as polyurethane fibers.

As the stretchable material of the comparatively weak straining force, all ways stretch tricot is particularly preferable, but the material is not limited to this and it is obvious that other materials can be used without hindering the purpose of this invention. Similarly, as a stretchable material of a comparatively strong straining force, for example, spandex power net is particularly preferable, but the material is not limited to this and it is obvious that other materials can be used without hindering the purpose of this invention.

These materials can vary depending on the purpose, the wearer's taste, the shape or size of the wearer's arms, and therefore are not particularly limited, but for the portion of a relatively weak straining force it is preferable to use a stretchable material having a straining force in the direction of the width of 0,294 - 0,784 N (30 - 80 gf) and for the portion of a relatively strong straining force it is preferable to use a stretchable material having a straining force in the direction of the width of 0,98 - 2,94 N (100 - 300 gf).

Stretching force toward length and straining force in the direction of the width may either be the same or different in these materials, but it is preferable that the materials have stretchability in both directions. The above mentioned numerical ranges of straining force are the straining force mainly in the direction of the width of the garment's each material part in the present invention, namely the material's straining force perpendicular to the length of the arm support garment in the present invention. More precisely, the materials do not necessarily lie exactly parallel to the length of the arm support garments as obviously seen in FIG. 1 (b) or FIGs. 8-10, and therefore, when measuring the straining force almost perpendicular to the length of a knit fabric or a woven fabric, it is preferable to measure along the direction of stitches or threads in the direction of the width. In the case the direction of stitches or threads is in the bias at almost an angle of 45' to the length and therefore it is difficult to judge the direction of stitches or threads because the material has two directions of +45' and -45' toward the length of the arm support garment and cannot be judged which one is width and which one is length or in other cases which are difficult to judge the direction of stitches or threads, the straining force is measured at an angle of about 90° to the length of the arm support garment. In the case the material to be measured has a layer structure of two or more materials (for example, said portion of a relatively strong straining force made of all ways stretch tricot having spandex power net lined thereto) will be measured as it is, namely with the layer structure.

The straining force in the direction of the width is emphasized because this straining force is more important than the straining force in the longitudinal direction. As mentioned before, it is preferable that the material has proper stretchability in the longitudinal direction for wearing convenience, but the stretchability can vary in a scope not hindering the purpose of this invention.

As the concrete method to measure stretchability, Constant-Rate of Specimen-Extension (CRE) type tensile tester ("AUTOGRAPH" AG-500D made by Shimadzu Corporation) is used to conduct stretch and recovery for three times at a tension speed of 300 ± 20 mm/min to 80% of the length of the specimen length (the clamping distance) and the load of the time of 30% stretch and the time of recovery of the third stretch and recovery are recorded and the average figure of the values of the two kinds is defined as the straining force. The size of the specimen is 2.5 cm in width and 10 cm in length. The straining force on the same sample may vary to some extent depending on the portion to measure or the straining force on samples made of the same material may vary to some extent due to the type of the dyestuff.

The thickness of a stretchable material of either a comparatively weak straining force or a comparatively strong straining force is preferably the same as general woven fabrics or knit fabrics, between 0.3 - 0.8 mm, because such thickness provides a garment which is thin, light, excellently fittable, having good ventilation, without interruption to physical movement nor reflecting unevenness to the upper garment to deteriorate the silhouette.

When a knit fabric or a woven fabric is used as a stretchable material of a comparatively weak straining force or a stretchable material of a comparatively strong straining force, such fabrics can be made of various kinds of synthetic fiber or natural fiber, in particular, it is preferable to combine with an elastic fiber such as polyurethane fiber to have better stretchability.

In the arm support garments of the present invention, it is possible to further equip articulation portions such as wrists with comparatively elastic plastic sheets such as elastomer resin or amorphous resin or sheets or bones of harder resin or metal material to reduce the chances of overextension or sprain.

The present invention provides an arm support garment which can relieve or prevent the muscle fatigue in forearm portion and consequestly prevent disorders caused by the accumulation of muscle fatigue factors such as lactic acid, can be worn easily and adequately by anybody, without blood circulation disorders nor nervous disorders, and enables the smooth movement of arms.

Since a preferable embodiment of the arm support garments of the present invention has a tube-like shape and comprises a stretchable material of a comparatively weak straining force in the whole region and a stretchable material of a comparatively strong straining force superimposed on the weak straining force material at the portion along the vicinity of the both longitudinal sides of said motor point of the flexor muscle group and/or the extensor muscle group extending almost along the longitudinal direction of forearms, the garment can be manufactured simply by superimposing a stretchable material of a relatively strong straining force onto specific portions of the garment comprised of a stretchable material of a relatively weak straining force without a complicated design or production process of seaming some pieces of fabrics of a stretchable material of a relatively weak straining force and some pieces of fabrics of a stretchable material of a relatively strong straining force alternately.

Since a preferable embodiment of the arm support garments of the present invention utilizes a stretchable knit fabric or a stretchable woven fabric as the stretchable material, a garment with thinness, lightness, better fittability, excellent ventilation, and comfortable feeling can be provided.

Since a preferable embodiment of the arm support garments of the present invention utilizes a stretchable material of a comparatively weak straining force in the direction of the width of 0,294 - 0,784 N (30 - 80 gf) and a stretchable material of a comparatively strong straining force in the direction of the width of 0,98 - 2,94 N (100 - 300 gf), the garment provides well-balanced functions of physical movements without interfering with the arms' movements, and relief and prevention of fatigue without blood circulation disorders. Moreover, a garment with soft fittability, better feeling and ventilation, easiness to put on and excellent fittability to the size of the wearer's arm can be provided.

In a preferable embodiment of the arm support garments in this invention using all ways stretch tricot as a stretchable material of a comparatively weak straining force, because the all ways stretch tricot is a thin knit fabric, a garment with lightness and soft fittability, better feeling and ventilation, easiness to put on due to its easy expansion, and excellent fittability regardless of the size of the arm of the wearer can be provided.

In a preferable embodiment of the arm support garments in the present invention using spandex power net as a stretchable material of a comparatively strong straining force unlike pile fabric or neoprene used to the conventional supporters, a garment with thinness, lightness, excellent fittability, without interruption of the arms' movements, good wearing silhouette, good ventilation without stuffiness, and sufficiently strong straining force on the specified portions can be provided.

Since a preferable embodiment of the arm support garments in this invention is further provided with a strap portion to insert fingers at the end of the wrist side, with an easy operation such as inserting fingers into the strap, a garment which can easily be worn on the proper portion and oriented in the appropriate direction, and which would not slide up toward the elbow even in sports activities and also which can follow the arm's twisting movements to allow the stretchable material to stay on the targeted muscle regions even when the wearer twists the arm and subsequently the muscle positions change can be provided.

In a preferable embodiment of the arm support garments in the present invention further provided with a protection piece in the vicinity of the wrist to prevent overextension, a garment which can further reduce the chances of overextension or sprain of the wrist can be provided.

## Claims

1. An arm support garment comprising a tube-like shaped stretchable material, wherein a stretchable material (7, 8, 9, 10) having a comparatively weak straining force is applied to the portion (8, 9) which in use covers motor points of each of the flexor muscle group and the extensor muscle group, extending substantially in the longitudinal direction of the user's forearm parts; and wherein a stretchable material (4, 5, 6) having a comparatively strong straining force is applied to the portion (8, 9), which is characterized in that it in use covers the vicinity of the both longitudinal sides of said motor points of the flexor muscle group (111, 112, 113) and the extensor muscle group (114, 115, 116).

2. The arm support garment of claim 1, wherein the garment comprises a tube-like shaped stretchable material (7, 8, 9, 10) of a comparatively weak straining force and a stretchable material (4, 5, 6) of a comparatively strong straining force superimposed onto the portion (8, 9) which in use covers the vicinity of the both longitudinal sides of the motor points of the flexor muscle group (111, 112, 113) and the extensor muscle group (114, 115, 116).

3. The arm support garment of claims 1 to 2, wherein the stretchable material is a stretchable knit fabric or a stretchable woven fabric.

4. The arm support garment of claims 1 to 2, wherein the Stretchable material (7, 8, 9, 10) of a comparatively weak straining force has a straining force in the direction of the width of 0,294 - 0,784 N (30 - 80 gf), and the stretchable material of a comparatively strong straining force has a straining force in the direction of the width of 0,98 - 2,94 N (100 - 300 gf).

5. The arm support garment of any one of claims 1 to 4, wherein the stretchable material (7, 8, 9, 10) with a comparatively weak straining force is all ways stretch tricot.

6. The arm support garment of any one of claims 1 to 5, wherein the stretchable material (4, 5, 6) with a comparatively strong straining force is spandex power net.

7. The arm support garment of any one of claims 1 to 6, wherein a strap portion (2) to insert fingers is provided on the end of the garment at the wrist side.

8. The arm support garment of any one of claims 1 to 7, wherein a protection piece to prevent overextension is further provided in the portion which in use is in the vicinity of the wrist.

## Patentansprüche

1. Armbandage, die ein röhrenartig geformtes dehnbares Material aufweist, wobei ein dehnbares Material (7,8,9,10) mit einer vergleichbar schwachen Verformungskraft auf den Abschnitt (8,9) angewendet wird, der im Gebrauch die motorischen Zentren sowohl der Beugemuskelgruppe als auch der Streckmuskelgruppe bedeckt, die sich im wesentlichen in Längsrichtung der Vorderarmabschnitte des Benutzers erstrecken; und wobei ein dehnbares Material (4,5,6) mit einer vergleichbar starken Verformungskraft auf den Abschnitt (8,9) angewendet wird, der dadurch gekennzeichnet ist, daß er im Gebrauch die Umgebung der beiden Längsseiten der motorischen Zentren der Beugemuskelgruppe (111,112,113) und der Streckmuskelgruppe (114,115,116) bedeckt,

2. Armbandage nach Anspruch 1, wobei die Bandage ein röhrenartig geformtes dehnbares Material (7,8,9,10) mit einer vergleichbar schwachen Verformungskraft und ein dehnbares Material (4,5,6) mit einer vergleichbar starken Verformungskraft aufweist, das über dem Abschnitt (8,9) liegt, der im Gebrauch die Umgebung der beiden Längsseiten der motorischen Zentren der Beugemuskelgruppe (111,112,113) und der Streckmuskelgruppe (114,115,116) bedeckt.

3. Armbandage nach Anspruch 1 bis 2, wobei das dehnbare Material ein dehnbarer Strickstoff oder ein dehnbarer Webstoff ist.

4. Armbandage nach Anspruch 1 bis 2, wobei das dehnbare Material (7,8,9,10) mit einer vergleichbar schwachen Verformungskraft eine Verformungskraft in Richtung der Breite von 0,294-0,784N (30-80gf) hat und das dehnbare Material mit einer vergleichbar starken Verformungskraft eine Verformungskraft in Richtung der Breite von 0,98-2,94N (100-300gf) hat.

5. Armbandage nach einem der Ansprüche 1 bis 4, wobei das dehnbare Material (7,8,9,10) mit einer vergleichbar schwachen Verformungskraft Allways-Stretchtricot-Stoff ist.

6. Armbandage nach einem der Ansprüche 1 bis 5, wobei das dehnbare Material (4,5,6) mit einer vergleichbar starken Verformungskraft Spandex-Power-Net-Stoff ist.

7. Armbandage nach einem der Ansprüche 1 bis 6, wobei an einem Ende der Bandage an der Handgelenksseite ein Riemenabschnitt (2) zum Einsetzen von Fingern vorgesehen ist.

8. Armbandage nach einem der Ansprüche 1 bis 7, wobei ferner in dem Abschnitt, der im Gebrauch in der Umgebung des Handgelenks ist, ein Schutzstück zum Verhindern einer Überdehnung vorgesehen ist.

## Revendications

1. Bandage pour bras comprenant un materiau extensible de forme tubulaire, dans lequel un matériau extensible (7, 8, 9, 10) développant une force de contrainte relativement faible est appliqué à la partie (8, 9) qui, lors de sa mise en oeuvre, recouvre les points moteurs de chacun du groupe de muscles fléchisseurs et du groupe de muscles extenseurs, s'étendant sensiblement selon la direction longitudinale des parties de l'avant-bras de l'utilisateur ; et dans lequel un matériau extensible (4,5,6) développant une force de contrainte relativement forte est appliqué à la partie (8, 9) qui est caractérisée en ce que, lors de sa mise en oeuvre, elle recouvre le voisinage des deux côtés longitudinaux desdits points moteurs du groupe de muscles fléchisseurs (111, 112, 113) et du groupe de muscles extenseurs (114, 115, 116).

2. Bandage pour bras selon la revendication 1, dans lequel le bandage comprend un matériau extensible de forme tubulaire (7, 8, 9, 10) développant une force de contrainte relativement faible et un matériau extensible (4,5,6) développant une force de contrainte relativement forte superposé à la partie (8, 9) qui, lors de sa mise en oeuvre, recouvre le voisinage des deux côtés longitudinaux desdits points moteurs du groupe de muscles fléchisseurs (111, 112, 113) et du groupe de muscles extenseurs (114, 115, 116).

3. Bandage pour bras selon la revendication 1 ou 2, dans lequel le matériau extensible est un tissu tricoté extensible ou un tissu extensible.

4. Bandage pour bras selon la revendication 1 ou 2, dans lequel le matériau extensible (7, 8, 9, 10) développant une force de contrainte relativement faible exerce une force de contrainte selon la direction de la largeur de 0,294-0,784 N (30 - 80 gf), et le matériau extensible développant une force de contrainte relativement forte exerce une force de contrainte selon la direction de la largeur de 0,98-2,94 N (100 - 300 gf).

5. Bandage pour bras selon l'une quelconque des revendications 1 à 4, dans lequel le matériau extensible (7, 8, 9, 10) développant une force de contrainte relativement faible est un tricot extensible dans toutes les directions.

6. Bandage pour bras selon l'une quelconque des revendications 1 à 5, dans lequel le matériau extensible (4, 5, 6) développant une force de contrainte relativement forte est un filet "spandex power".

7. Bandage pour bras selon l'une quelconque des revendications 1 à 6, dans lequel une partie de bande (2) destinée à l'insertion des doigts est prévue à l'extrémité du bandage, du côté du poignet.

8. Bandage pour bras selon l'une quelconque des revendications 1 à 7, dans lequel une pièce de protection destinée à empêcher une surextension est, en outre, prévue dans la partie qui, lors de sa mise en oeuvre, se trouve au voisinage du poignet.
